# EUROPÄISCHE PATENTANMELDUNG

(11) **EP 0 826 693 A1**
(43) Veröffentlichungstag der Anmeldung: **04.03.1998**
(21) Anmeldenummer: 97114436.5
(22) Anmeldetag: 21.08.1997
(51) Int. Cl.: C07F 9/54, C07C 39/02

(54) **Verfahren zur Herstellung von Phosponiumphenolaten**

(30) Priorität: 03.09.1996 DE 19635656; 05.06.1997 DE 19723524
(71) Anmelder: BAYER AG, 51368 Leverkusen (DE)
(72) Erfinder: König, Annett, Dr., 47800 Krefeld (DE); Kühling, Steffen, Dr., 40670 Meerbusch (DE); Bunzel, Lothar, 47906 Kempen (DE); Hucks, Uwe, Dipl.-Ing., 46519 Alphen (DE)

(57) **Zusammenfassung**

Gegenstand der vorliegenden Erfindung ist ein Verfahren zur Herstellung von Phosphoniumphenolaten aus Phosphoniumhalogeniden und Phenolen.

## Beschreibung

Gegenstand der vorliegenden Erfindung ist ein Verfahren zur Herstellung von Phosphoniumphenolaten, das dadurch gekennzeichnet ist, daß man Phosphoniumhalogenide mit Phenolen in einem Gemisch aus Wasser, wäßriger Alkalilauge und inertem Lösungsmittel zwischen 2 Minuten und 4 Stunden, vorzugsweise zwischen 20 Minuten und 2 Stunden bei Temperaturen von 0°C bis 40°C, vorzugsweise von 15°C bis 30°C, und bei Drücken von 1 bar bis 20 bar, vorzugsweise von 1 bar bis 10 bar im Molverhältnis von 0,5 mol bis 2 mol, vorzugsweise von 0,7 mol bis 1,3 mol Phenol, pro Mol Phosphoniumhalogenid, umsetzt.

Die Molverhältnisse Phenol/Alkali sind von 10:8 bis 10:10, bevorzugt 10:9,5 bis 10:10, besonders bevorzugt von 10:9,9.

Phosphoniumphenolate eignen sich besonders als Katalysatoren zur Veresterung und zur Umesterung, insbesondere zur Herstellung von Polycarbonaten nach dem Schmelzumesterungsverfahren (siehe US-PS 3 442 854).

Bevorzugte Phosphoniumhalogenide sind solche der Formel (I) 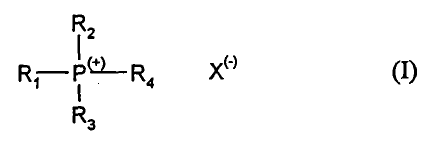 worin
- R₁ bis R₄: unabhängig voneinander C₁-C₁₂-Alkyl, C₅-C₆-Cycloalkyl, C₇-C₁₂-Aralkyl und C₆-C₁₄-Aryl sind und worin
- X^{⊖}: Halogen, vorzugsweise F^{⊖}, Cl^{⊖} oder Br^{⊖} ist.

Bevorzugte Reste R₁ bis R₄ sind C₆-C₁₄-Aryl-Reste.

Derartige Phosphoniumhalogenide und ihre Herstellung sind bekannt (siehe beispielsweise "Houben-Weyl, Methoden der organischen Chemie" Band XII/1, Seiten 79 ff. und Worrall, J. Amer. Chem. Soc. 52 (1930), Seiten 293 ff.).

Diese Verbindungen (I) entstehen bei der Umsetzung von Trialkyl- oder Triarylphosphinen, beispielsweise von Triphenylphosphin mit Halogenarylen oder Halogenalkylen, z.B. Benzylbromid in Gegenwart von Metallsalzen (Friedel-Crafts-Alkylierung) oder in Gegenwart von Grignard-Verbindungen und Kobalt(II)-chlorid.

Bevorzugte Phenole sind die der Formel (II) 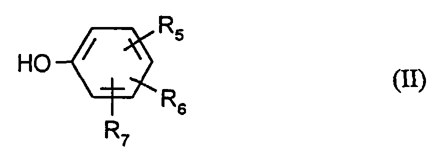 worin
- R₅ bis R₇: unabhängig voneinander H, C₁-C₁₂-Alkyl, C₅-C₆-Cycloalkyl, C₇-C₁₂-Arylalkyl und C₆-C₁₄-Aryl sind; vorzugsweise sind R₅ bis R₇ H.

Derartige Phenole sind literaturbekannt.

Bevorzugte Phosphoniumphenolate sind somit solche der Formel (III) 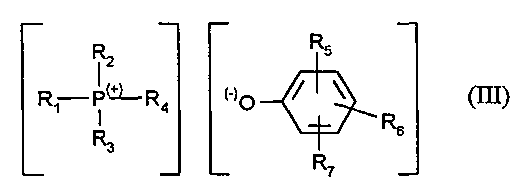 worin die Reste R₁ bis R₇ denen der Formeln (I) und (II) entsprechen.

Bevorzugtes Wasser ist 3E-Wasser bzw. destilliertes Wasser. Bevorzugte Alkalilaugen sind Natronlauge und Kalilauge.

Die Konzentration der Alkalilaugen liegt zwischen 3 und 20, besonders bevorzugt zwischen 8 und 12 Gew.-%, bezogen auf die wäßrige Phase.

Bevorzugte inerte Lösungsmittel sind CH₂Cl₂, und Cl-C₆H₅.

Die Volumenverhältnisse von wäßriger Phase zu inertem Lösungsmittel liegen zwischen 2:1 und 1:1 Volumenteile. Die Konzentration an Phosphoniumhalogeniden in der flüssigen Phase liegt vor der

Umsetzung zwischen 10 und 20 Gew.-%, vorzugsweise zwischen 12 und 15 Gew.-%.

Die Konzentration an Phenol liegt vor der Umsetzung zwischen 20 und 30 Gew.-%, bezogen auf Gewicht wäßrige Phase und inertes Lösungsmittel.

Die Reaktion kann kontinuierlich oder diskontinuierlich erfolgen, vorzugsweise erfolgt sie diskontinuierlich.

Die Isolierung der erfindungsgemäßen Phosphoniumphenolate erfolgt durch 3-maliges Waschen mit 3E- bzw. destilliertem Wasser, anschließender Eindampfung der organischen Phase am Rotationsverdampfer und Trocknung bei 80°C im Vakuum-Trockenschrank.

Die Ausbeuten liegen zwischen 70 und 80 Gew.-%.

Erfindungsgemäß hergestellte quartäre Phosphoniumphenolate sind beispielsweise

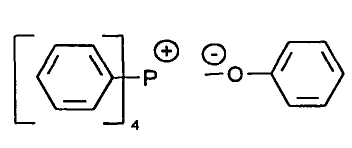

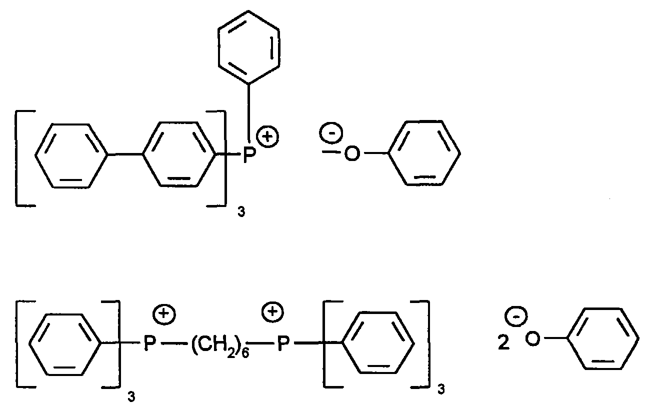

Das erfindungsgemäße Verfahren war deshalb nicht naheliegend, weil der Austausch der Halogenide gegen andere Anionen, wie Hydroxid, Phenolat in wäßriger Lösung durch Umsetzung mit dem entsprechenden Salz nicht gelingt, da die Phosphoniumsalze durch Basen in das entsprechende Phosphinoxid überführt werden.

So entsteht bei der Umsetzung von Tetraphenylphosphoniumbromid mit Phenol in wäßriger Natronlauge Triphenylphosphinoxid (siehe Beispiel A). Auch die Umsetzung von Tetraphenylphosphoniumbromid mit Silberoxid in Wasser zur freien Base (Hydroxid) geht weiter bis zum Triphenylphosphinoxid und Benzol.

Es bestand daher die Aufgabe, eine gute Methode zur Herstellung von quartären Phosphoniumphenolaten zu finden, um sie beispielsweise als Umesterungskatalysatoren zur Herstellung von thermoplastischen, aromatischen Polycarbonaten einzusetzen, denn sie sind zum einen besser einsetzbar und enthalten zum anderen weniger Fremdatome (z.B. kein Bor) als andere Phosphoniumkatalysatoren, beispielsweise als Tetraphenylphosphonium-tetraphenylboranat, und sind besser als beispielsweise Natriumphenolat.

Daß zudem nach dem erfindungsgemäßen Zweiphasengrenzflächenverfahren die Herstellung der Phosphoniumphenolate mit guter Ausbeute erfolgt, war nicht zu erwarten.

Die Verwendung der erfindungsgemäß erhältlichen Phosphoniumphenolate zur Herstellung von aromatischen Polycarbonaten kann in an sich bekannter Weise erfolgen (siehe US-PS 3 442 854 loc. cit.).

Das Schmelzumesterungsverfahren geht bekanntlich beispielsweise aus von aromatischen Diphenolen, Kohlensäurediarylestern und gegebenenfalls Verzweigern und/oder Monophenolen.

Die erfindungsgemäß erhältlichen Phosphoniumphenolate werden hierbei als Katalysatoren in Mengen von 10⁻¹ mol bis 10⁻⁸ mol, vorzugsweise in Mengen von 10⁻³ mol bis 10⁻⁷ mol, pro mol Diphenol eingesetzt.

Weitere Einzelheiten des Schmelzumesterungsverfahrens sind in der Literatur beschrieben (siehe beispielsweise Hermann Schnell, Chemistry and Physics of Polycarbonates, Polymer Reviews, Vol. 9, 1964, Seiten 44 bis 51, DE-AS 1 031 512, US-PS 3 022 272, US-PS 5 340 905 und US-PS 5 399 659).

Die mit den erfindungsgemäß erhältlichen Phosphoniumphenolaten hergestellten thermoplastischen Polycarbonate sind lösungsmittelfrei, mit heller Eigenfarbe ausgestattet und weitgehend frei von unerwünschten Fehlstellen im Polycarbonat.

Der technische Einsatz dieser so hergestellten Polycarbonate in Form der verschiedensten Formteile ist überall dort möglich, wo bislang bereits thermoplastische Polycarbonate eingesetzt wurden, etwa in der Elektrotechnik, als Lampenabdeckungen, als Sicherheitsscheiben oder als CD-Material.

### Beispiel A

### Herstellung von Phosphoniumphenolaten in alkalischer Lösung

In einem 2,5 l-Rundkolben mit Rührer, Thermometer, Rückflußkühler und Tropftrichter werden 94,0 g (1,0 mol) Phenol, 88,0 g (0,99 mol) 45 %ige Natronlauge und 1,5 l 3E-Wasser vorgelegt und bei 20°C bis 25°C gerührt. Der pH-Wert dieser Lösung ist etwa 14. Zu dieser Lösung werden 41,93 g (0,10 mol) Tetraphenylphosphoniumbromid zugegeben. Anschließend wird mindestens 0,5 h gerührt. Die phenolische Lösung wird 3 mal mit 3E-Wasser gewaschen und anschließend eingedampft. Der kristalline Rückstand wird bei 100°C im Vakuum getrocknet.

### Beispiel B

### Herstellung von Phosphoniumphenolaten im Zweiphasengrenzflächenverfahren

In einem 2,5 l-Rundkolben mit Rührer, Thermometer und Tropftrichter werden 94,0 g (1,0 mol) Phenol, 88,0 g (0,99 mol) 45 %ige Natronlauge und 1,5 l 3E-Wasser vorgelegt und bei 20°C bis 25°C gerührt. Zu dieser Lösung werden 41,93 g (0,10 mol) Tetraphenylphosphoniumbromid, gelöst in 100 ml Methylenchlorid, zugegeben. Anschließend wird mindestens 0,5 h gerührt. Nach der Phasentrennung wird die organische Phase 3 mal mit 3E-Wasser gewaschen und anschließend eingedampft. Der kristalline Rückstand wird bei 100°C im Vakuum getrocknet.

Zusammensetzung (aus ¹H-NMR):

| | Beispiel A | Beispiel B |
|---|---|---|
| Substanz | mol Teile | |
| Tetraphenylphosphonium-Kation | 17,2 | 25,9 |
| Triphenylphosphinoxid | 35,3 | 0,4 |
| Phenol/Phenolat | 47,5 | 73,7 |

### Beispiel C

### Verwendungsbeispiele

C1) In einem 500 ml Dreihalskolben mit Rührer, Innenthermometer und Vigreuxkolonne (30 cm, verspiegelt) mit Brücke werden 114,15 g (0,500 mol) Bisphenol A und 113,54 g (0,530 mol) Diphenylcarbonat eingewogen. Die Apparatur wird durch Anlegen von Vakuum und Spülen mit Stickstoff (3 mal) vom Luftsauerstoff befreit und das Gemisch auf 150°C aufgeheizt. Jetzt werden 0,00216 g (1*10⁻³ mol-%) Tetraphenylphosphoniumphenolat (TPP-P), bezogen auf Bisphenol A, als 1 %ige wäßrige Lösung zugegeben und das entstehende Phenol bei 100 mbar abdestilliert. Gleichzeitig wird die Temperatur bis auf 250°C gesteigert. Nun wird das Vakuum stufenweise bis auf 1 mbar verbessert und die Temperatur auf 260°C erhöht. Anschließend wird die Temperatur auf 280°C erhöht und bei 0,1 mbar 1,5 Stunden gerührt. Man erhält ein farbhelles, lösungsmittelfreies Polycarbonat mit einer relativen Lösungsviskosität von 1,250 (Dichlormethan, 25°C, 5 g/l).
   Der Gehalt an Verzweiger der Formel (IV) im hergestellten Polycarbonat beträgt 25 ppm. Der phenolische OH-Wert des Polycarbonats beträgt 70 ppm. 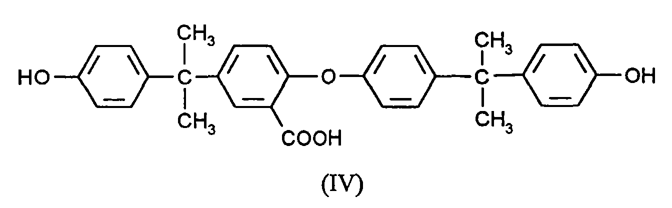
C2) Wie Beispiel C1), nur wird die Temperatur von 260°C auf 300°C erhöht und bei 0,1 mbar 1,5 Stunden gerührt. Man erhält ein farbhelles, lösungsmittelfreies Polycarbonat mit einer relativen Lösungsviskosität von 1,300 (Dichlormethan, 25°C, 5 g/l). Der Gehalt an Verzweiger der Formel (IV) im hergestellten Polycarbonat beträgt 18 ppm. Der phenolische OH-Wert des Polycarbonats beträgt 55 ppm.
C3) Wie Beispiel C1), nur wird die Temperatur von 260°C auf 320°C erhöht und bei 0,1 mbar 1,5 Stunden gerührt. Man erhält ein farbhelles, lösungsmittelfreies Polycarbonat mit einer relativen Lösungsviskosität von 1,392 (Dichlormethan, 25°C, 5 g/l). Der Gehalt an Verzweiger der Formel (IV) im hergestellten Polycarbonat beträgt 23 ppm. Der phenolische OH-Wert des Polycarbonats beträgt 60 ppm.

### Beispiel D

### Verwendungsbeispiele (Vergleich)

### Vergleichsbeispiel D1)

In einem 500 ml Dreihalskolben mit Rührer, Innenthermometer und Vigreuxkolonne (30 cm, verspiegelt) mit Brücke werden 114,15 g (0,500 mol) Bisphenol A und 113,54 g (0,530 mol) Diphenylcarbonat eingewogen. Die Apparatur wird durch Anlegen von Vakuum und Spülen mit Stickstoff (3 mal) vom Luftsauerstoff befreit und das Gemisch auf 150°C aufgeheizt. Jetzt werden 0,00029 g (5*10⁻⁴ mol-%) Natriumphenolat, bezogen auf Bisphenol A, als 1 %ige wäßrige Lösung zugegeben und das entstehende Phenol bei 100 mbar abdestilliert. Gleichzeitig wird die Temperatur bis auf 250°C gesteigert. Nach 1 Stunde wird das Vakuum auf 10 mbar verbessert. Durch Absenken des Vakuums auf 0,5 mbar und Temperaturerhöhung auf 280°C wird die Polykondensation erreicht. Man erhält ein lösungsmittelfreies Polycarbonat mit einer relativen Lösungsviskosität von 1,288 (Dichlormethan, 25°C, 5 g/l). Der Gehalt an Verzweiger der Formel (IV) im hergestellten Polycarbonat beträgt 358 ppm. Der phenolische OH-Wert des Polycarbonats beträgt 95 ppm.

## Patentansprüche

1. Verfahren zur Herstellung von Phosphoniumphenolaten, dadurch gekennzeichnet, daß man Phosphoniumhalogenide mit Phenolen in einem Gemisch aus Wasser, wäßriger Alkalilauge und inertem Lösungsmittel zwischen 2 Minuten und 4 Stunden bei Temperaturen von 0°C bis 40°C und bei Drücken von 1 bar bis 20 bar im Molverhältnis von 0,5 mol bis 2 mol Phenol, pro Mol Phosphoniumhalogenid, umsetzt.

2. Verfahren gemäß Anspruch 1, dadurch gekennzeichnet, daß man im Molverhältnis von 0,7 mol bis 1,3 mol Phenol pro Mol Phosponiumhalogenid, umsetzt.
